# EUROPÄISCHE PATENTSCHRIFT

(11) **EP 0 942 895 B1**
(45) Veröffentlichungstag und Bekanntmachung des Hinweises auf die Patenterteilung: **31.10.2001**
(21) Anmeldenummer: 97940141.1
(22) Anmeldetag: 29.08.1997
(51) Int. Cl.: C07C 41/00, C07C 43/00

(54) **VERFAHREN ZUR HERSTELLUNG VON ALKOXYBUTENEN**
PROCESS FOR PRODUCING ALKOXYBUTENES
PROCEDE POUR LA PREPARATION D'ALCOXYBUTENES

(30) Priorität: 17.09.1996 DE 19637892
(43) Veröffentlichungstag der Anmeldung: 22.09.1999
(73) Patentinhaber: BASF AKTIENGESELLSCHAFT, 67056 Ludwigshafen (DE)
(72) Erfinder: KANAND, Jürgen, D-67098 Bad Dürkheim (DE); RÖPER, Michael, D-67157 Wachenheim (DE)
(86) Internationale Anmeldenummer: EP9704704
(87) Internationale Veröffentlichungsnummer: WO9812165

(56) Entgegenhaltungen:
- EP-A- 0 025 240
- DE-A- 2 550 902
- DE-A- 4 400 837
- GB-A- 943 160
- US-A- 2 922 822

## Beschreibung

Die vorliegende Erfindung betrifft ein verbessertes Verfahren zur Herstellung von Alkoxybutenen durch Addition von Alkoholen an Butadien in Gegenwart von Wasser.

1-Alkoxybut-2-ene sind gesuchte Zwischenprodukte z.B. für die Herstellung von n-Butyraldehyd und n-Butanol gemäß dem in WO 95-19 334 beschriebenen Verfahren.

Es ist aus US 2 922 822 und DE-A 25 50 902 bekannt, daß Alkohole in flüssiger Phase mit 1,3-Butadien in Gegenwart von sauren Ionenaustauschern zu den entsprechenden ungesättigten Ethern reagieren. In US 2 922 822 wird diese Umsetzung in Gegenwart eines großen Überschusses an Methanol durchgeführt, was zu einer erhöhten Bildung des unerwünschten Dimethylethers führt. Gemäß dem Verfahren von DE-A 25 50 902 entsteht bei dieser Umsetzung Vinylcyclohexen als Hauptprodukt. Gemäß EP-A 25 240 wird die Addition von Alkoholen an 1,3-Butadien vorteilhaft in Gegenwart eines polaren, aprotischen Lösungsmittels vorgenommen, das dann wieder abdestilliert werden muß. Gemäß GB-A 943 160 wird die Addition von Alkoholen mit Brönsted-Säuren in Gegenwart von Kupfersalzen durchgeführt. Vor allem in WO 95-19 334 wird die Addition von Alkoholen an Butadien oder Butadien enthaltende Gemische mit bereits guten Ausbeuten eingehend beschrieben. (Dort ist auch beschrieben, wie man durch Isomerisierung von 3-Alkoxybut-1-enen allein die 1-Alkoxybut-2-ene herstellen kann.)

Dennoch bilden sich immer eine Vielzahl von Dimeren oder oligomeren Derivaten des Butadiens oder Dialkylether, für die es praktisch keine Verwendung gibt oder die nur in so geringen Mengen Anwendung finden, daß der größte Teil dieser als Zwangsanfall in einem großtechnischen Verfahren entstehenden Nebenprodukte entsorgt werden müßte.

Der vorliegenden Erfindung lag somit die Aufgabe zugrunde, eine weitere Verbesserung des Verfahrens zur Herstellung von Alkoxybutenen durch Addition von Alkoholen an Butadien zu finden, die es ermöglicht, diese Produkte mit gesteigerter Selektivität und Ausbeute herzustellen.

Diese Aufgabe wurde gelöst mit einem Verfahren zur Herstellung von Alkoxybutenen, bei dem 1,3-Butadien oder ein Butadien-haltiges Kohlenwasserstoffgemisch mit einem Alkohol der Formel ROH I bei erhöhter Temperatur und bei erhöhtem Druck in Gegenwart einer Brönsted-Säure zu einem Gemisch der Addukte der Formeln II und III umgesetzt wird, in der der Rest R eine unsubstituierte oder mit 1 bis 2 C₁-bis C₁₀-Alkoxy- oder Hydroxygruppen substituierte C₂-bis C₂₀-Alkyl- oder Alkenyl-, Cycloalkyl-, Cycloalkenyl, eine C₆- bis C₁₀-Aryl- oder eine C₇- bis C₁₁-Aralkylgruppe oder die Methylgruppe ist, 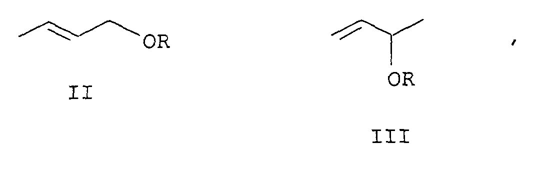 wobei die Verbesserung darin besteht, daß man die Reaktion in Gegenwart von Wasser, z.B. bis zu 20 Gew.-% Wasser, bezogen auf das flüssige Reaktionsgemisch, durchführt und die Brönstedtsäure ein organischer Kationenaustauscher in seiner protonierten Form ist.

### Im folgenden wird das erfindungsgemäße Verfahren näher erläutert:

In der Reaktion wird 1,3-Butadien oder ein Butadien-haltiges Kohlenwasserstoffgemisch in Gegenwart von Wasser und einer Brönsted-Säure mit dem Alkohol ROH I gemäß der Gleichung 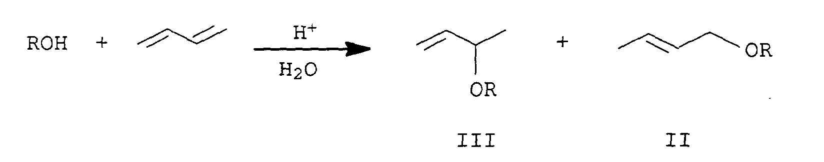 zum 1,4-Addukt der Formel II und dem 1,2-Addukt der Formel III umgesetzt. Im entstandenen 1,4-Addukt II kann die Doppelbindung sowohl in der cis- als auch trans-Form vorliegen. Die Addukte II und III entstehen dabei je nach Reaktionsbedingungen und angewandtem Katalysator im allgemeinen in einem Molverhältnis von 1:1 bis 1:3.

Die Art des in die Umsetzung eingesetzten Alkohols ROH I ist in der Regel für das Verfahren nicht kritisch. Es können sowohl primäre als auch sekundäre Alkohole verwendet werden, bevorzugt werden aber primäre Alkohole eingesetzt. Es können sowohl aliphatische, cycloaliphatische, aromatische als auch araliphatische Alkohole benutzt werden, vorzugsweise finden aliphatische und araliphatische Alkohole Anwendung. Im allgemeinen werden Alkohole ROH I im erfindungsgemäßen Verfahren verwendet, in denen der Rest R eine C₁- bis C₂₀-Alkyl-, C₂- bis C₁₀-Alkenyl-, z.B. die But-2-enyl-, vorzugsweise eine C₁ bis C₄-Alkyl-, insbesondere die n-Butylgruppe, eine C₁- bis C₂₀-Cycloalkyl- oder Cycloalkenyl-, eine C₆- bis C₁₀-Aryl-, vorzugsweise die Phenylgruppe oder eine C₇- bis C₁₁-Aralkyl-, vorzugsweise die Benzylgruppe ist. Die Reste R können gegebenenfalls mit Substituenten wie C₁- bis C₁₀-Alkoxy-und/oder Hydroxyl-Gruppen substituiert sein. Als Alkohole ROH I können somit auch Diole oder Triole oder Alkoxyalkohole verwendet werden. Da diese Substituenten in der Regel keinen kritischen Einfluß auf die Umsetzung haben, werden vorzugsweise Alkohole ROH I mit unsubstituierten Resten R verwendet. Selbstverständlich können auch Alkohole mit einer höheren Anzahl an Kohlenstoffatomen eingesetzt werden. Da solche höheren Alkohole in der Regel teurer sind als niedere Alkohole, werden aus wirtschaftlichen Gründen vor allem niedere Alkanole und insbesondere Butanol bevorzugt verwendet.

Als Brönsted-Säuren zur Addition von Alkoholen ROH I an 1,3-Butadien oder Butadien-haltigen Kohlenwasserstoffgemischen werden feste Brönsted-Säuren, insbesondere organische Kationentauscher, eingesetzt.

Unter organischen Kationenaustauschern werden pulverförmige, gelartige oder makroporöse, polymere Polyelektrolyte verstanden, die Brönsted-acide funktionelle Gruppen, wie Sulfonsäure-, Phosphonsäure- oder Carboxylgruppen auf einer polymeren Matrix tragen, beispielsweise sulfonierte Phenol-Formaldehyd-Harze, sulfonierte Styrol-Divinylbenzol-Copolymere, sulfoniertes Polystyrol, Poly(perfluoralkylen)sulfonsäuren oder sulfonierte Kohlen. Diese Kationenaustauscher können in Form handelsüblicher Produkte eingesetzt werden, wie sie unter den Handelsnamen Amberlite®, Dowex®, Amberlyst®, Lewatit®, Wofatit®, Permutit®, Purolite® und Nafion® im Handel erhältlich sind. Zweckmäßigerweise kommen die Kationenaustauscher in ihrer protonierten Form, der sogenannten H⁺-Form zur Anwendung. Als geeignete organische Kationenaustauscher seien beispielhaft die Handelsprodukte Amberlite® 200, Amberlite® IR 120, Amberlite® IR 132 E, Lewatit® SC 102, Lewatit® SC 104, Lewatit® SC 108, Lewatit® SPC 108, Lewatit® SPC 112, Lewatit® SPC 118, Purolite® CT 175, Purolite® CT 171, Amberlyst® CSP 2 und Amberlyst® 15 genannt.

Diese werden vorzugsweise in einem Festbett angeordnet und von der flüssigen Reaktionsmischung in der Sumpf- oder Rieselfahrweise durchströmt. Das Katalysatorfestbett kann z.B. in Rohrreaktoren oder vorzugsweise in Reaktorkaskaden eingebaut werden. Es ist auch möglich, die Reaktanten gasförmig durch die Katalysatorschüttung zu leiten, bevorzugt wird in der flüssigen Phase gearbeitet. Es versteht sich von selbst, daß die Addition des Alkohols ROH an 1,3-Butadien oder Butadien-haltigen Kohlenwasserstoffgemischen sowohl kontinuierlich als auch diskontinuierlich durchgeführt werden kann.

Das Molverhältnis Alkohol/1,3-Butadien kann aus einem weiten Bereich gewählt werden. Im allgemeinen wird ein Molverhältnis Alkohol ROH/1,3-Butadien von 0,5:1 bis 5:1 vorzugsweise von 1:1 bis 2,5:1 und besonders bevorzugt von 1,5:1 bis 2,5:1 angewandt. Die Umsetzung des Alkohols ROH I mit 1,3-Butadien oder Butadien-haltigen Kohlenwasserstoffgemischen wird im allgemeinen bei Temperaturen von 20 bis 150°C, vorzugsweise von 50 bis 120°C, insbesondere von 70 bis 110°C und bei einem Druck von im allgemeinen 1 bis 100 bar, vorzugsweise von 3 bis 50 bar, insbesondere von 5 bis 20 bar, vorgenommen. Die Anwendung eines höheren Drucks ist möglich. Die angewandte Reaktionstemperatur wird zweckmäßigerweise hinsichtlich des jeweils verwendeten Brönsted-Säure-Katalysators in einem Vorversuch optimiert.

Im allgemeinen wird das Alkohol ROH/1,3-Butadien-Gemisch mit einer Raumgeschwindigkeit von 0,01 bis 0,5 g/cm³·h, vorzugsweise von 0,02 bis 0,4 g/cm³·h und besonders bevorzugt von 0,02 bis 0,05 g/cm³·h durch das Katalysatorfestbett geleitet. Der Zusatz eines Lösungsmittels zum Reaktionsgemisch ist möglich, im allgemeinen aber nicht erforderlich, da der eingesetzte Alkohol als auch die Addukte II und III auch als Lösungsmittel fungieren können. Die Verweilzeit des Alkohol ROH/1,3-Butadiengemisches im Reaktor beträgt im allgemeinen 1 bis 6 Stunden und ist in der Regel von der angewandten Reaktionstemperatur abhängig.

Anstelle von reinem 1,3-Butadien können auch 1,3-Butadien-haltige Kohlenwasserstoffgemische als Rohstoff eingesetzt werden. Derartige Kohlenwasserstoffströme fallen beispielsweise als sogenannter C₄-Schnitt in Steamcrackern an. Zweckmäßigerweise werden diese Kohlenwasserstoffströme vor ihrem Einsatz von gegebenenfalls darin enthaltenen acetylenischen oder allenischen Kohlenwasserstoffen durch deren partielle Hydrierung (Weissermel, Arpe: Industrielle Organische Chemie; 3. Auflage, VCH Verlagsgesellschaft, Weinheim 1988) befreit. Die 1,3-Butadien-haltigen Kohlenwasserstoffströme können sodann in analoger Weise wie reines 1,3-Butadien eingetragen werden. Zweckmäßigerweise werden die in diesen Kohlenwasserstoffströmen enthaltenen gesättigten oder monoolefinischen Kohlenwasserstoffe, die bei der Umsetzung nicht reagiert haben, aus dem Reaktionsaustrag entfernt, beispielsweise mittels eines Gas-Flüssigkeit-Abscheiders.

Es hat sich nun überraschenderweise gezeigt, daß bei Zusatz von Wasser eine Selektivitäts-, und damit auch zu einer Ausbeutesteigerung erzielt wird.

Zur Herstellung der Alkoxybutene wird der Reaktionsmischung Wasser in einem Gewichtsanteil im allgemeinen zwischen 20 Gew.-% und 0,0001 Gew.-%, vorzugsweise zwischen 10 und 0,001 Gew.-% und besonders bevorzugt zwischen 5 und 0,01 Gew.-% bezogen auf das flüssige Reaktionsgemisch zugesetzt. Bei der diskontinuierlichen Betriebsweise kann das Wasser gemeinsam mit den anderen Reaktanten im Reaktor vorgelegt werden, es kann aber auch vorteilhaft sein, das Wasser erst nach dem Beginn der Umsetzung in den Reaktor zu dosieren. Welche dieser Verfahrensweisen letztlich gewählt wird, hängt vom jeweils verwendeten Katalysator und den angewandten Druck- und Temperaturbedingungen ab. Zweckmäßigerweise wird die optimale Verfahrensweise für den jeweils verwendeten Katalysator in einem Vorversuch ermittelt. In analoger Weise kann bei der kontinuierlichen Ausgestaltung des Verfahrens, z.B. in einem Rohr- oder Kaskadenreaktor, das Wasser gemeinsam mit den übrigen Reaktanten in den Reaktor geleitet oder aber erst nach einer gewissen Verweilzeit der Reaktanten im Reaktor, über einen separaten Einlaß in den Reaktor dosiert werden. In einer weiteren Ausgestaltung des erfindungsgemäßen Verfahrens kann das Wasser anstatt über eine Zudosierung in das Reaktionsgemisch auch über die Verwendung von wasserfeuchten Ionenaustauschern in das Reaktionsgemisch eingetragen werden und der Wasseranteil wenn nötig, durch Zudosierung von frischem Wasser oder Entfernung einer bestimmten Wassermenge erhöht bzw. erniedrigt werden.

### Beispiele

### Beispiel 1 (Reaktion ohne Wasser)

Ein 0,3 1 Rührautoklav wurde mit 65,7 g (0,89 mol) n-Butanol und mit 15,0 g Purolite® CT 175 in der H⁺-Form, der vorher mit Wasser und Aceton gewaschen und im Stickstoffstrom getrocknet worden war, befüllt. Anschließend wurden 33,2 g (0,61 mol) 1,3-Butadien in den Reaktor gepreßt. Nach 10 h Reaktionszeit bei einer Temperatur von 90°C und 9 bar Druck wurde bei einem Umsatz von 73 % eine Selektivität von 40,8 % an 3-Butoxybut-1-en, 40,9 % an 1-Butoxybut-2-en, 11,7 % an Butoxyoctadienen und 3,4 % an Dibutylether erhalten, das ist eine Gesamtausbeute an Alkoxybutenen von 59,6 %.

### Beispiel 2 (Reaktion in Gegenwart von Wasser)

Ein 0,3 1 Rührautoklav wurde mit 65,7 g (0,89 mol) n-Butanol, 1,2 g Wasser und mit 15,0 g Purolite® CT 175 in der H⁺-Form, der vorher mit Wasser und Aceton gewaschen und im Stickstoffstrom getrocknet worden war, befüllt. Anschließend wurden 38,0 g (0,70 mol) 1,3-Butadien in den Reaktor gepreßt. (Die von Beispiel 1 abweichende Menge Butadien ist zufällig, da beim Aufpressen keine exakte Dosierung möglich ist. Die geringfügig andere Menge beeinflußt die Ausbeute nicht.) Nach 10 h Reaktionszeit bei einer Temperatur von 90°C und 9 bar Druck wurde bei einem Umsatz von 71 % eine Selektivität von 45,2 % an 3-Butoxybut-1-en, 42,5 % an 1-Butoxybut-2-en, 5,9% an Butoxyoctadienen und 1,1 % an Dibutylether erhalten, das ist eine Gesamtausbeute an Dialkoxybutenen von 62,3 %.

## Patentansprüche

1. Verfahren zur Herstellung von Alkoxybutenen, durch Umsetzung von 1,3-Butadien oder eines Butadien-haltigen Kohlenwasserstoffgemisches mit einem Alkohol der Formel ROH I bei erhöhter Temperatur und bei erhöhtem Druck in Gegenwart einer Brönsted-Säure zu einem Gemisch der Addukte der Formeln II und III 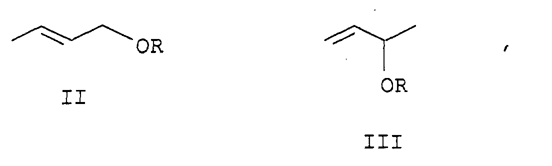 in der der Rest R eine unsubstituierte oder mit 1 bis 2 C₁-bis C₁₀-Alkoxy- oder Hydroxygruppen substituierte C₂- bis C₂₀-Alkyl-, Alkenyl-, Cycloalkyl oder Cycloalkenyl oder eine C₂-bis C₁₀-Aryl- oder eine C₇- bis C₁₁-Aralkylgruppe oder die Methylgruppe ist, **dadurch gekennzeichnet, daß** man die Reaktion in Gegenwart von Wasser durchführt und die Brönsted-Säure ein organischer Kationenaustauscher in seiner protonierten Form ist.

2. Verfahren nach Anspruch 1, **dadurch gekennzeichnet, daß** man die Umsetzung in Gegenwart von bis zu 20 Gew.-% Wasser, bezogen auf das flüssige Reaktionsgemisch, vornimmt.

3. Verfahren nach Anspruch 1, **dadurch gekennzeichnet, daß** man die Umsetzung in Gegenwart von 0,001 bis 10 Gew.-% Wasser, bezogen auf das flüssige Reaktionsgemisch, vornimmt.

4. Verfahren nach Anspruch 1, **dadurch gekennzeichnet, daß** man die Umsetzung in Gegenwart von 0,01 bis 5 Gew.-% Wasser, bezogen auf das flüssige Reaktionsgemisch, vornimmt.

5. Verfahren nach Anspruch 1, **dadurch gekennzeichnet, daß** man als Alkohol ROH I n-Butanol verwendet.

## Claims

1. A process for preparing alkoxybutenes by reacting 1,3-butadiene or a butadiene-containing hydrocarbon mixture with an alcohol of the formula ROH I at elevated temperature and at elevated pressure in the presence of a Brönsted acid to give a mixture of the adducts of the formulae II and III 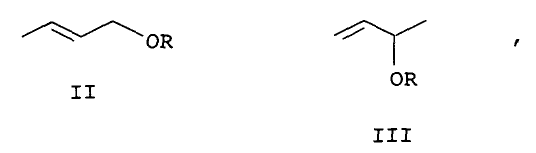 where the radical R is a C₂-C₂₀-alkyl, alkenyl, cycloalkyl or cycloalkenyl group, each of which may be unsubstituted or substituted by 1 or 2 C₁-C₁₀-alkoxy or hydroxy groups, or a C₂-C₁₀-aryl group or a C₇-C₁₁-aralkyl group or the methyl group, wherein the reaction is carried out in the presence of water and the Brönsted acid is an organic cation exchanger in its protonated form.

2. A process as claimed in claim 1, wherein the reaction is carried out in the presence of up to 20 % by weight of water, based on the liquid reaction mixture.

3. A process as claimed in claim 1, wherein the reaction is carried out in the presence of from 0.001 to 10 % by weight of water, based on the liquid reaction mixture.

4. A process as claimed in claim 1, wherein the reaction is carried out in the presence of from 0.01 to 5 % by weight of water, based on the liquid reaction mixture.

5. A process as claimed in claim 1, wherein the alcohol ROH I used is n-butanol.

## Revendications

1. Procédé de préparation des alcoxybutènes au moyen de la réaction entre le 1,3-butadiène ou bien un mélange d'hydrocarbures contenant du butadiène, avec un alcool de formule ROH I à une température élevée et sous une pression élevée en présence d'un acide de Brönsted, obtenant ainsi un mélange de produits d'addition de formule II et III 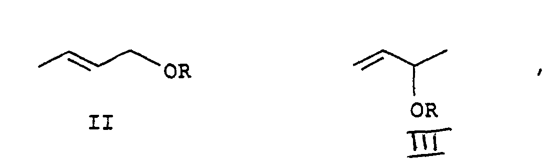 dans lesquelles le résidu R est un groupe alcényle, cycloalkyle, cycloalcényle ou alkyle en C₂ à C₂₀, non substitué ou substitué par 1 à 2 groupes alkoxy en C₁ à C₁₀ ou hydroxyle, ou bien un groupe aryle en C₂ à C₁₀ ou un groupe aralkyle en C₇ à C₁₁ ou le groupe méthyle, **caractérisé en ce que** l'on réalise la réaction en présence d'eau, et que l'acide de Brönsted est un échangeur de cations organique sous sa forme protoné.

2. Procédé selon la revendication 1, **caractérisé en ce que** l'on réalise la réaction en présence d'eau, en une quantité allant jusqu'à 20% en poids par rapport au mélange réactionnel liquide.

3. Procédé selon la revendication 1, **caractérisé en ce que** l'on réalise la réaction en présence d'eau, à raison de 0,001 à 10% en poids par rapport au mélange réactionnel liquide.

4. Procédé selon la revendication 1, **caractérisé en ce que** l'on réalise la réaction en présence d'eau, à raison de 0,01 à 5% en poids par rapport au mélange réactionnel liquide.

5. Procédé selon la revendication 1, **caractérisé en ce que** l'on met en oeuvre, en tant qu'alcool ROH I, le n-butanol.
